# EUROPÄISCHE PATENTSCHRIFT

(11) **EP 1 915 183 B1**
(45) Veröffentlichungstag und Bekanntmachung des Hinweises auf die Patenterteilung: **27.11.2013**
(21) Anmeldenummer: 06763017.8
(22) Anmeldetag: 09.08.2006
(51) Int. Cl.: A61L 29/08, A61L 31/08

(54) **VERWENDUNG VON NICHTIONISCHEN ESTERN IN EINER BESCHICHTUNG FÜR MIT BLUT IN KONTAKT KOMMENDE OBERFLÄCHEN**
USE OF NONIONIC ESTERS IN A COATING FOR SURFACES ENTERING IN CONTACT WITH BLOOD
UTILISATION D'ESTERS NON IONIQUES DANS UN REVETEMENT DE SURFACES DESTINEES A ENTRER EN CONTACT AVEC DU SANG

(30) Priorität: 16.08.2005 DE 102005040211
(43) Veröffentlichungstag der Anmeldung: 30.04.2008
(73) Patentinhaber: Maquet Cardiopulmonary AG, 76437 Rastatt (DE)
(72) Erfinder: NAKEL, Mathias, 72393 Burladingen/Ringingen (DE); EISENLOHR, Birgit, 72379 Hechingen (DE)
(74) Vertreter: Laufer, Gabriele
(86) Internationale Anmeldenummer: PCT/EP2006/007867
(87) Internationale Veröffentlichungsnummer: WO 2007/019994

(56) Entgegenhaltungen:
- EP-A1- 0 309 345
- EP-A2- 0 404 515
- WO-A-86/02933
- WO-A-03/094991
- WO-A1-01/23015
- WO-A1-2005/004946
- WO-A2-00/50106
- DE-A1- 10 221 055
- US-A1- 2001 003 796
- HIGUCHI A ET AL: "Serum protein adsorption and platelet adhesion on pluronic@?-adsorbed polysulfone membranes" BIOMATERIALS, ELSEVIER SCIENCE PUBLISHERS BV., BARKING, GB, Bd. 24, Nr. 19, August 2003 (2003-08), Seiten 3235-3245, XP004425368 ISSN: 0142-9612
- HENNEUSE-BOXUS C ET AL: "Surface functionalization of PEEK films using photochemical routes" EUROPEAN POLYMER JOURNAL, PERGAMON PRESS LTD. OXFORD, GB, Bd. 37, Nr. 1, Januar 2001 (2001-01), Seiten 9-18, XP004217935 ISSN: 0014-3057
- GELDERBLOM H ET AL: "Cremophor EL - the drawbacks and advantages of vehicle selection for drug formulation" EUROPEAN JOURNAL OF CANCER, PERGAMON PRESS, OXFORD, GB, Bd. 37, Nr. 13, September 2001 (2001-09), Seiten 1590-1598, XP004301125 ISSN: 0959-8049

## Beschreibung

Die vorliegende Erfindung betrifft die Verwendung von zumindest einem nichtionischen Ester in einer Beschichtung für mit Blut in Kontakt kommende Oberflächen zur Verbesserung der Hämokompatibilität der Oberflächen.

Die Erfindung betrifft ferner eine medizinische Vorrichtung mit zumindest einer Oberfläche, die eine Beschichtung aufweist, in welcher ein nichtionischer Ester verwendet wird.

Im Stand der Technik sind verschiedene Beschichtungszusammensetzungen und Verfahren zur Beschichtung von medizinischen Vorrichtungen bekannt.

Bei vielen medizinischen Behandlungen werden Kunststoffoberflächen verwendet, die über längere oder kürzere Zeiträume mit dem Blut eines Patienten in Kontakt kommen. Bei derartigen Vorrichtungen handelt es sich bspw. um Einmalgeräte für eine Herz-Lungen-Maschine, Oxygenatoren, Katheter, künstliche Organe wie Herz oder Niere, Gasaustauschmembranen oder vaskuläre Prothesen, wobei diese Aufzählung keineswegs als abschließende Aufzählung zu verstehen ist.

Bei diesen Kunststoffoberflächen ist es u.a. von äußerster Wichtigkeit, die Koagulation des mit den Oberflächen in Kontakt kommenden Blutes zu verhindern, und insgesamt die Oberfläche hämokompatibel zu gestalten.

In diesem Zusammenhang ist es bspw. bekannt, die Blutgerinnung durch eine hochdosiert Gabe von Heparin zu hemmen oder aber Heparin bzw. Heparin derivate an diejenigen Oberflächen zu binden, die mit Blut in Kontakt kommen (siehe z.B. WO 03/094991 A1).

Ferner ist es im Stand der Technik bekannt, Oberflächen mit hydrophilen Polymeren oder Tensiden wie bspw. Pluronic™ zu beschichten, von welchem gezeigt werden konnte, dass es die Hämokompatibilität von mit Blut in Kontakt kommenden Oberflächen verbessert. So werden bspw. in der US 6,670,199 verschiedene Beschichtungen beschrieben, die als Grund-Gerüst Pluronic™ aufweisen, das mit unterschiedlichen Biomolekülen konjugiert sein kann.

Versuche im Hause der Anmelderin haben nun überraschend gezeigt, dass die o.g. Beschichtungen zwar antithrombogene Eigenschaften aufweisen, dass diese aber gleichzeitig auch eine starke Verschlechterung der Komplementaktivierung zeigten. Die bekannten Beschichtungen entsprechen damit in diesem Punkt nicht dem, was unter guter Blutverträglichkeit zu verstehen ist.

Gute Blutverträglichkeit (= Hämokompatibilität) bedeutet für eine Oberfläche nämlich, dass sie bei Kontakt mit Blut weder die Blutgerinnung noch die Abwehrmechanismen des Körpers gegen die Fremdoberfläche anstößt.

Gerade die bei der bekannten Beschichtung mit Pluronic™ überraschenderweise gefundene hohe Komplementaktivierung ist besonders von Nachteil, da sie zu systemischen Entzündungen führen und bspw. postoperatives Organversagen verursachen kann.

Im Stand der Technik sind ferner Beschichtungen mit hämokompatiblen Polymeren beschrieben. Diese setzen jedoch die Verwendung von geeigneten organischen Lösungmitteln voraus. Die zu beschichtenden Kunststoffe können durch diese Lösungsmittel aber in unzulässiger Weise angegriffen werden, wodurch die Funktion der Vorrichtungen beeinträchtigt werden kann. Polymere mit geringerem Molekulargewicht, aber ausreichender Löslichkeit, um aus wässrigen Medien verabreicht werden zu können, zeigen eine höhere Tendenz, durch den Blutstrom von der Oberfläche abgewaschen zu werden.

Vor diesem Hintergrund liegt der vorliegenden Erfindung die Aufgabe zugrunde, alternative Substanzen bereitzustellen, welche zur Beschichtung für mit Blut in Kontakt kommende Oberflächen verwendet werden können, um die Hämokompatibilität derart beschichteter Oberflächen zu verbessern und die mit einem einfachen Prozess aus wässrigen Medien als Lösung oder Emulsion aufgebracht werden können, aber dennoch eine ausreichende Haftung aufweisen, um nicht durch den Blutstrom abgewaschen zu werden

Diese Aufgabe wird durch die Bereitstellung eines nichtionischen Esters gelöst, wobei der nichtionische Ester aus einem acyclischen C₃-C₆(OH)₃-₆ Polyol und aus zumindest drei C₁₂-C₂₆ Fettsäuren gebildet ist, und wobei der nichtionische Ester ferner zumindest eine hydrophile Gruppe aufweist.

Die der Erfindung zugrundeliegende Aufgabe wird auf diese Weise vollkommen gelöst.

Die EP 0 535 485 A1 offenbart einen Filterpad, der mit einer Lösungszusammensetzung imprägniert wird, welche Cremophor® EL enthalten kann. Der Filterpad wird eingesetzt, um Vollblutproben bzw. dessen zelluläre Bestandteile aufzutrennen.

Die US 2001/0003796 A1 offenbart eine zweilagige Beschichtung für mit Blut in Kontakt kommende Oberflächen von medizinischen Vorrichtungen wie bspw. Stents oder Ballonkatheter. Die Unterschicht kann dabei Cremophor® aufweisen, und dient der Verbesserung der Gleit-/Schmierfähigkeit.

Die WO 01/23015 A1 offenbart die Verwendung von Cremophor^{®} EL zur Beschichtung von Oberflächen um damit die Reibung zu reduzieren.

Die WO 2005/004946 A1 offenbart die Verwendung von Cremophor^{®} EL zur Beschichtung von Oberflächen um damit später freizusetzende Wirkstoffe in unveränderter Form zu bewahren bzw. zu stabilisieren.

Die Erfinder der vorliegenden Anmeldung haben nämlich überraschenderweise festgestellt, dass durch die Verwendung der genannten nichtionischen Ester insgesamt die Hämokompatibilität von mit Blut in Kontakt kommenden Oberflächen verbessert wird. Im Vergleich mit Pluronic™ beschichteten Oberflächen konnten insbesondere hinsichtlich der Komplementaktivierung große Verbesserungen erzielt werden.

Die Erfinder haben beispielhaft verschiedene Substanzen, die ein erfindungsgemäß definiertes Grundgerüst aufweisen, sowohl im HLM(Herz-Lungen-Maschine)-*in-vitro-*Test als auch im Chandler-Loop-Test verschiedenen Tests hinsichtlich Hämokompatibilität unterzogen. In eigenen Versuchen konnte gezeigt werden, dass mit der neuen Beschichtung, u.a. im Test auf Anzahl und Aktivierung von Blutplättchen (β-Thromboglobulin), eine deutliche Verbesserung der Hämokompatibilität im Vergleich zu unbeschichteten und mit Pluronic™ beschichteten Oberflächen erzielt werden konnte.

Vorteilhaft gegenüber bereits bekannten Beschichtungen ist ferner, dass mit den neu bereitgestellten Substanzen eine optimale Haftung der Beschichtung an der mit Blut in Kontakt kommenden Oberfläche erzielt werden kann. Eine gute Haftung der Beschichtung an der Oberfläche ist von besonderer Wichtigkeit, da ungenügend haftende Substanzen zum einen in den Blutstrom gelangen können und im Patienten dann ev. Nebenwirkungen auslösen können. Andererseits besteht wiederum bei Beschichtungen, die ungenügend haften und durch den Blutstrom abgewaschen werden, die Gefahr, dass die Oberfläche keine ausreichende Hämokompatibilität mehr aufweist und es - neben den durch die freigesetzte Beschichtungssubstanz ausgelösten Nebenwirkungen - bspw. zusätzlich zu einer Aktivierung der Blutkomponenten durch Kontakt mit der bloßgelegten Oberfläche kommen kann.

Bei der vorliegend erstmals bereitgestellten Beschichtungssubstanz, also dem nicht-ionischen Ester, ist durch die zumindest 3 Fettsäuren gewährleistet, dass die Substanz fest an der Oberfläche haftet, bzw. an der Oberfläche verankert wird. Ein nachteiliges Abwaschen mit den oben beschriebenen möglichen Folgen wird dadurch erfolgreich vermieden.

Ferner wird durch den hydrophilen Anteil des nichtionischen Esters die antithrombogene Eigenschaft der Beschichtung und damit deren gute Hämokompatibilität gewährleistet.

Gemäß einer Weiterbildung der erfindungsgemäßen Verwendung kann die hydrophile Gruppe des Esters ausgewählt sein aus der Gruppe umfassend Hydroxyl, Methoxyl, Ethoxyl und Ethoxylat, Homopolymere von Vinylverbindungen und Copolymere von Vinylverbindungen. Dabei ist insbesondere bevorzugt, wenn die Vinylverbindung ausgewählt ist aus der Gruppe umfassend Vinylpyrrolidon, Acrylamid, Acrylsäureester, Methacrylsäureester.

Die Erfinder der vorliegenden Anmeldung haben erkannt, dass die hydrophile Gruppe des Esters aus den genannten Gruppen ausgewählt werden kann, da die aufgezählten Gruppen hinreichend hydrophil sind, was insgesamt für eine optimale Hämokompatibilität wichtig ist. Wie bereits oben erwähnt, bildet das acyclische Polyol mit den Fettsäuren dabei einen Anker, über welchen die Substanz an der Oberfläche dauerhaft haften kann. Die hydrophilen Gruppen sorgen für die antithrombogene Wirkung der Beschichtung.

In einer Ausführungsform der erfindungsgemäßen Verwendung ist bevorzugt, wenn die hydrophile Gruppe über das Polyol mit dem nichtionischen Ester verbunden ist.

Bei dieser Ausführungsform ist also die zumindest eine hydrophile Gruppe über das C₃-C₆(OH)₃₋₆ Polyol mit dem Ester verknüpft. Dadurch wird bei einer gleichzeitigen Gewährleistung der stabilen Verankerung der Beschichtung auf der Oberfläche wiederum gleichzeitig für eine ausreichende hydrophile Eigenschaft der Beschichtung gesorgt.

In einer anderen Ausführungsform der erfindungsgemäßen Verwendung ist bevorzugt, wenn die hydrophile Gruppe über die Fettsäure mit dem Ester verbunden ist.

Auch diese Ausführungsform bietet die Vorteile einer festen Verankerung der Beschichtung auf der Oberfläche bei gleichzeitig guten hydrophilen Eigenschaften. Auch hier sorgen das acyclische Polyol und die Fettsäuren für eine stabile Verankerung, wohingegen wiederum der hydrophile Anteil, der diesmal über die Fettsäuren mit dem Ester verbunden ist, für eine optimale Hämokompatibilität sorgt. Bspw. weist bei dieser Ausführungsform die erfindungsgemäß verwendete Substanz Fettsäuren auf, von denen mindestens eine eine Hydroxylgruppe besitzt, die ethoxyliert ist.

In einer weiteren Ausführungsform ist bevorzugt, wenn der nichtionische Ester eine Formel aufweist, die aus den folgenden Formeln ausgewählt ist: 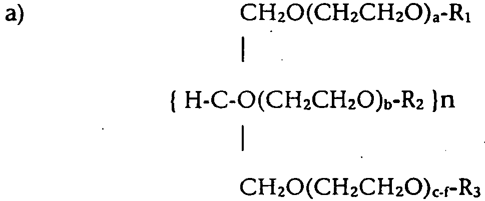 in welcher
R₁₋₃ jeweils eine C₁₂ - C₂₆ Fettsäure ist, die gleich oder unterschiedlich, gesättigt oder ungesättigt sind und die ggf. zumindest eine Hydroxylgruppe aufweisen,
n eine ganze Zahl von 1 bis 4 ist, und
a-f ganze Zahlen sind, die gleich oder unterschiedlich sein können, wobei die Summe der Zahlen a-f zwischen 0 und 200 liegt; 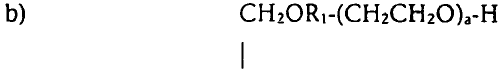 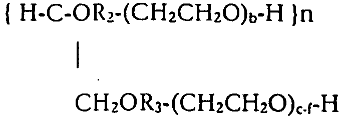
in welcher
R₁₋₃ jeweils eine C₁₂ - C₂₆ Fettsäure ist, die gleich oder unterschiedlich, und gesättigt oder ungesättigt sind, wobei zumindest einer der Reste₁₋₃ zumindest eine Hydroxylgruppe aufweist,
n eine ganze Zahl von 1 bis 4 ist, und
a-f ganze Zahlen sind, die gleich oder unterschiedlich sein können, wobei die Summe der Zahlen a-f zwischen 0 und 200 liegt; 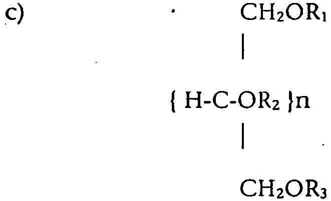
in welcher
R₁₋₃ jeweils eine C₁₂ - C₂₆ Fettsäure ist, die gleich oder unterschiedlich, und gesättigt oder ungesättigt sind, wobei an zumindest einen der Reste R₁₋₃ mindestens ein hydrophiler Rest aus Homo- oder Copolymeren von Vinylverbindungen geknüpft ist,
n eine ganze Zahl von 1 bis 4 ist.

Es versteht sich, dass der zumindest eine hydrophile Rest bei der Synthese der Verbindung über zumindest eine Doppelbindung der Reste R₁₋₃ mit diesen verknüpft wird.

Die genannten Alternativen bieten aufgrund des gemeinsamen Grundgerüsts sämtlich den Vorteil, dass eine stabile Verankerung der Substanz auf der Oberfläche sowie eine für eine gute Hämokompatibilität ausreichende hydrophile Eigenschaft gewährleistet ist.

Die Erfinder konnten in eigenen Versuchen zeigen, dass eine erfindungsgemäß verwendete Substanz, nämlich das im Stand der Technik als Lösungsvermittler/Emulgator verwendete Cremophor EL, den damit beschichteten Oberflächen eine ausgezeichnete Hämokompatibilität vermittelt. Cremophor stellt dabei ein Beispiel für einen ethoxylierten nichtionischen Ester aus acyclischem Polyol mit Fettsäuren dar. Getestet wurde die genannte Substanz bspw. hinsichtlich der Plättchenanzahl und Aktivierung (β-Thromboglobulin) usw. im Vergleich zu im Stand der Technik bekannten Substanzen.

Durch diese Versuche konnten die Erfinder ableiten, dass ähnlich aufgebaute Substanzen ebenfalls für eine optimale hämokompatible Beschichtung geeignet sind. In einer weiteren Ausführungsform ist bevorzugt, wenn der nichtionische Ester eine Formel aufweist die aus den folgenden Formeln ausgewählt ist: 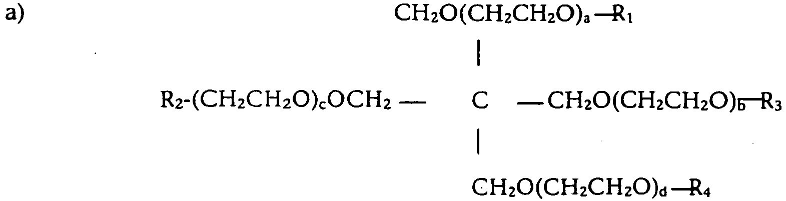 in welcher
R₁₋₄ jeweils eine C₁₂-C₂₆ Fettsäure ist, die gleich oder unterschiedlich, gesättigt oder ungesättigt sind und die ggf. zumindest eine Hydroxylgruppe aufweisen, und
a-d ganze Zahlen sind, die gleich oder unterschiedlich sein können, wobei die Summe der Zahlen a-d zwischen 0 und 200 liegt; 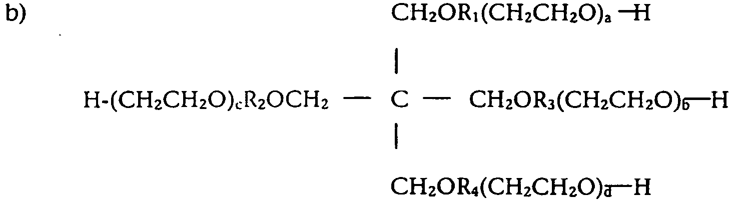
in welcher
R₁₋₄ jeweils eine C₁₂-C₇₆ Fettsäure ist die gleich oder unterschiedlich, gesättigt oder ungesättigt sind, wobei zumindest einer der Reste R₁₋₄ zumindest eine Hydroxylgruppe aufweist,
und
a-d ganze Zahlen sind, die gleich oder unterschiedlich sein können, wobei die Summe der Zahlen a-d zwischen 0 und 200 liegt; 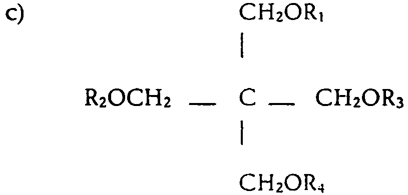
in welcher
R₁₋₄ jeweils eine C₁₂-C₂₆ Fettsäure ist, die gleich oder unterschiedlich, gesättigt oder ungesättigt sind, wobei zumindest an einen der Reste R₁₋₄ mindestens ein hydrophiler Rest aus Homo- oder Copolymeren von Vinylverbindungen geknüpft ist.

Auch hier versteht sich, dass der zumindest eine hydrophile Rest bei der Synthese der Verbindung über zumindest eine Doppelbindung der Reste R₁₋₃ mit diesen verknüpft wird.

Die Erfinder konnten auf Grund eigener Versuche ableiten, dass mit diesen Verbindungen, welche verzweigte Moleküle auf Basis von Pentaerythrit darstellen, eine ebenso gute hämokompatible Eigenschaft von Beschichtungen erzielt werden kann wie die o.g. unverzweigten Moleküle. Fettsäureester von Pentaerythrit werden im Stand der Technik u.a. als Emulgatoren eingesetzt.

Insgesamt ist bevorzugt, wenn der nichtionische Ester einen HLB-Wert (HLB = hydrophilic-lipophilic balance) von zwischen 2 und 18, vorzugsweise zwischen 4 und 14 aufweist.

Der HLB-Wert beschreibt das Verhältnis zwischen hydrophilen und lipophilen Anteilen einer chemischen Verbindung. Die Erfinder haben festgestellt, dass durch die Einstellung des HLB-Werts auf die genannten Werte eine Substanz bereitgestellt werden kann, die der Beschichtung eine optimal angepasste hydrophile Eigenschaft verleiht.

In einer weiteren Ausführungsform ist bevorzugt, wenn der nichtionische Ester ausgewählt ist aus der Gruppe umfassend Polyoxylglycerol-Ricinoleat, Polyoxylglyerol-hydroxystearat, Polyoxylsorbitolhexaoleat.

Diese Verbindungen haben sich in eigenen Versuchen als geeignet für die Beschichtung im Sinne der vorliegenden Anmeldung bewiesen. Ein Beispiel für Polyoxylglycerol-Ricinoleat (Synonym: Macrogol-glycerolricinoleat) ist Cremophor® EL, ein Beispiel für Polyoxylglycerol-hydroxystearat (Synonym: Macrogol-glycerolhydroxystearat) ist Cremophor® WO 7, und ein Beispiel für Polyoxylsorbitolhexaoleat (Synonym: Macrogolsorbitolhexaoleat) ist Atlas® G 1086 oder Atlas® G 1096. Die genannten Substanzen sind im Handel kommerziell erhältlich.

Die Aufzählungen sind nur beispielhaft, da alle denkbaren Substanzen, die eine erfindungsgemäße Struktur aufweisen, eingesetzt werden können.

Es ist ferner bevorzugt, wenn die Beschichtungslösung für die hämokompatible Beschichtung aus einer wässrigen Lösung oder Emulsion besteht, die den nichtionischen Ester enthält.

Allgemein ist bevorzugt, wenn der nichtionische Ester in der wässrigen Lösung oder Emulsion zu weniger als 2 Gew.-%, vorzugsweise zu weniger als 0,2 Gew.-% enthalten ist.

Die Erfinder der vorliegenden Anmeldung haben erkannt, dass bereits diese Anteile des nichtionischen Esters in der Lösung zu einer deutlichen Verbesserung der hämokompatiblen Eigenschaften führen.

Vor diesem Hintergrund offenbart die vorliegende Erfindung ferner eine hämokompatible Beschichtung für Oberflächen, die mit Blut in Berührung kommen, in welcher zumindest ein nichtionischer Ester wie zuvor definiert verwendet wird.

Mit der hämokompatiblen Beschichtung können also - wie bereits erwähnt - Oberflächen von bspw. medizinischen Vorrichtungen beschichtet werden, die mit Blut in Kontakt kommen, und bei welchen es von großer Wichtigkeit ist, dass diese gute hämokompatible Eigenschaften erhalten. Dadurch wird verhindert, dass bspw. das Komplementsystem im Blut aktiviert wird, oder dass bspw. Blutplättchen oder weiße Blutkörperchen adsorbiert bzw. aktiviert werden.

Ferner offenbart die vorliegende Erfindung eine medizinische Vorrichtung mit zumindest einer Oberfläche, die die offenbarte hämokompatible Beschichtung aufweist. Dabei kann die Oberfläche der medizinischen Vorrichtung eine Kunststoffoberfläche sein, insbesondere aus Polypropylen, Polycarbonat, Polymethylpenten, Polyurethan, Polyethylen, Polyester, Silikon, Hart- oder Weich-Polyvinylchlorid, Copolymere wie z.B. ABS, EPDM usw.

Auch kann die medizinische Vorrichtung ein Bestandteil einer mit Blut in Kontakt kommenden Vorrichtung sein, vorzugsweise von Einmalgeräten einer Herz-Lungen-Maschine, eines Oxygenators, eines Katheters, eines künstlichen Herzens, einer künstlichen Niere, einer Gasaustauschmembran oder einer vaskulären Prothese.

Weitere Vorteile ergeben sich aus der beigefügten Beschreibung und der Tabelle.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, sofern sie in den Schutzbereich der angefügten Ansprüche fallen.

**Tabelle 1: Ergebnisse der Chandler-Loop-Tests**

| | Kontrolle | Unbeschichtet | Pluronic F68 | Cremophor EL |
|---|---|---|---|---|
| Blutplättchen x 10⁻³/µl | 264 | 187 | 225 | 249 |
| ß-TG IU/ml | 72,2 | 1601,0 | 1760,9 | 809,0 |
| SC5b-9 ng/ml | 156,3 | 1864,7 | 7577 | 3148 |

In der Tabelle 1 sind Ergebnisse von unterschiedlich beschichteten Probekörpern dargestellt, die einem Test im Chandler-Loop-Modell unterzogen wurden. Dabei rotieren teilweise mit Blut gefüllte Schläuche (Loops) mit verschiedenen Beschichtungen im Wasserbad. Dadurch steht die "Blutsäule", und der Schlauch rotiert um die Blutsäule und simuliert einen Fluss. Nach 90 Minuten wurden den Loops Blut entnommen und diese Proben auf bestimmte Werte analysiert.

Die Probekörper (Loops) bestanden in den vorliegend durchgeführten Tests aus 7 handelsüblichen Konnektoren aus Polycarbonat, die durch 3/8" Silikonschlauchstücke zu einem Ring verbunden wurden.

Je 5 solcher Loops wurden zur Beschichtung zusammengesteckt und 1000 ml wässrige Beschichtungslösung unter Verwendung von 1 m 3/8" Pumpschlauch aus Silikon mit einer Schlauchpumpe bei einem Fluss von zwei Liter pro Minute für 20 Minuten bei Raumtemperatur im Kreislauf umgepumpt. Nach der Beschichtung wurde die Beschichtungslösung verworfen und die beschichteten Loops ohne weitere Spülung mit steriler Pressluft ausgeblasen und dadurch getrocknet. Nach einer Endtrocknung von vier Stunden bei 40°C im Trockenschrank war der Beschichtungsprozess beendet. Die verwendeten Beschichtungslösungen wiesen folgende Zusammensetzungen auf: Beschichtung A: 1000 mg/Liter Cremophor®EL (Caesar + Loretz GmbH, Hilden) in demineralisiertem Wasser; Beschichtung B: 1000 mg/Liter Pluronic®F68 (AppliChem GmbH, Darmstadt) in demineralisiertem Wasser.

In Tabelle 1 wird die Anzahl der Blutplättchen nach Versuchsdurchführung für jeden getesteten Schlauch aufgeführt. Der Tabelle ist zu entnehmen, dass die Anzahl der Blutplättchen bei der Cremophor-Beschichtung geringfügig höher als bei der Pluronic-Beschichtung, jedoch deutlich höher als bei der unbeschichteten Kontrolle liegt.

In Tabelle 1 ist außerdem das Ergebnis der β-Thromboglobulinbestimmung dargestellt (quantitativ, IU/ml). β-Thromboglobulin wird bei der Aktivierung von Blutplättchen ausgeschüttet, so dass die Konzentration von β-Thromboglobulin ein Maß für die Aktivierung der Blutplättchen ist.

Wie der Tabelle 1 zu entnehmen ist, führt die Cremophor-Beschichtung zu einer deutlich geringeren Ausschüttung von β-Thromboglobulin als die mit Pluronic™ beschichtete und die unbeschichtete Oberfläche.

In der Tabelle 1 ist zusätzlich das Ergebnis der Bestimmung des SC5b-9-Komplements (Nanogramm/ml) dargestellt. Der terminale Komplement-Komplex ist ein relevanter Marker für die Evaluation der Hämokompatibilität von Oberflächen. SC5b-9 besitzt die Fähigkeit, Zellmembranen zu attackieren und bspw. Blutplättchen zu aktivieren. Daher muss es bei jeder Beschichtung Ziel sein, die Bildung dieses Komplementkomplexes so gering wie möglich zu halten.

Der Tabelle 1 ist wieder im Vergleich zwischen Pluronic™ und Cremophor zu entnehmen, dass Cremophor zu einem weitaus niedrigeren SC5b-9-Wert führt als Pluronic®F68 (ca. 3000 ng/ml für Cremophor im Vergleich zu ca. 7600 ng/ml für Pluronic; jeweils Mittelwerte).

Mit den Ergebnissen im Chandler-Loop zeigt sich, dass die erfindungsgemäße Beschichtung gegenüber der aufgeführten Beschichtung aus dem Stand der Technik bessere hämokompatible Eigenschaften aufweist.

## Patentansprüche

1. Verwendung von zumindest einem nichtionischen Ester in einer Beschichtung für mit Blut in Kontakt kommende Oberflächen zur Verbesserung der Hämokompatibiltät der Oberflächen, wobei der nichtionische Ester aus einem acyclischen C₃ - C₆ (OH)₃₋₆ Polyol und aus zumindest drei C₁₂- C₂₆ Fettsäuren gebildet ist, wobei der Ester ferner zumindest eine hydrophile Gruppe aufweist.

2. Verwendung nach Anspruch 1, **dadurch gekennzeichnet, dass** die hydrophile Gruppe des Esters ausgewählt ist aus der Gruppe umfassend Hydroxyl, Methoxyl, Ethoxyl und Ethoxylat, Homopolymere von Vinylverbindungen und Copolymere von Vinylverbindungen.

3. Verwendung nach Anspruch 1 oder 2, **dadurch gekennzeichnet, dass** die Vinylverbindung ausgewählt ist aus der Gruppe umfassend Vinylpyrrolidon, Acrylamid, Acrylsäureester, Methacrylsäureester.

4. Verwendung nach einem der Ansprüche 1 bis 3, **dadurch gekennzeichnet, dass** die zumindest eine hydrophile Gruppe über das Polyol mit dem Ester verbunden ist.

5. Verwendung nach einem der Ansprüche 1 bis 3, **dadurch gekennzeichnet, dass** die zumindest eine hydrophile Gruppe über die Fettsäure mit dem Ester verbunden ist.

6. Verwendung nach einem der Ansprüche 1 bis 3, **dadurch gekennzeichnet, dass** der nichtionische Ester eine Formel aufweist, die aus den folgenden Formeln ausgewählt ist: 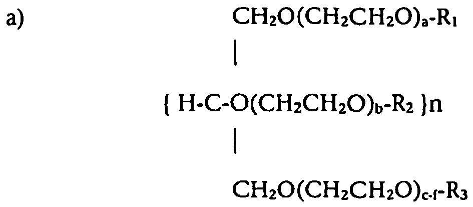 in welcher
R₁₋₃ jeweils eine C₁₂ - C₂₆ Fettsäure ist, die gleich oder unterschiedlich, gesättigt oder ungesättigt sind und die ggf. zumindest eine Hydroxylgruppe aufweisen,
n eine ganze Zahl von 1 bis 4 ist, und
a-f ganze Zahlen sind, die gleich oder unterschiedlich sein können, wobei die Summe der Zahlen a-f zwischen 0 und 200 liegt. 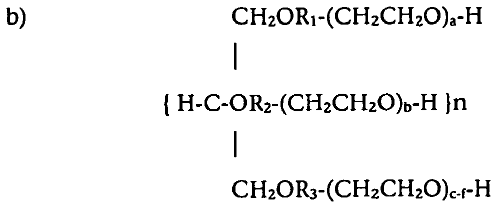
in welcher
R₁₋₃ jeweils eine C₁₂ - C₂₆ Fettsäure ist, die gleich oder unterschiedlich, und gesättigt oder ungesättigt sind, wobei zumindest einer der Reste₁₋₃ zumindest eine Hydroxylgruppe aufweist,
n eine ganze Zahl von 1 bis 4 ist, und
a-f ganze Zahlen sind, die gleich oder unterschiedlich sein können, wobei die Summe der Zahlen a-f zwischen 0 und 200 liegt. 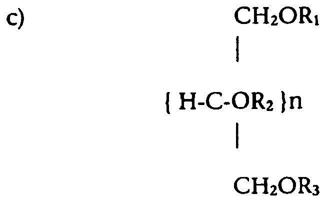
in welcher
R₁₋₃ jeweils eine C₁₂ - C₂₆ Fettsäure ist, die gleich oder unterschiedlich, und gesättigt oder ungesättigt sind, wobei zumindest an einen der Reste R₁₋₃ mindestens ein hydrophiler Rest aus Homo- oder Copolymeren von Vinylverbindungen geknüpft ist,
n eine ganze Zahl von 1 bis 4 ist.

7. Verwendung nach einem der Ansprüche 1 bis 3, **dadurch gekennzeichnet, dass** der nichtionische Ester eine Formel aufweist, die aus den folgenden Formeln ausgewählt ist: 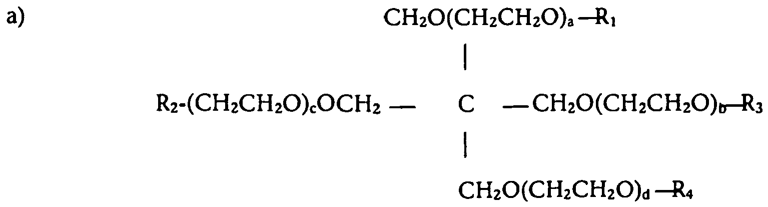 in welcher
R₁₋₄ jeweils eine C₁₂-C₂₆ Fettsäure ist, die gleich oder unterschiedlich, gesättigt oder ungesättigt sind und die ggf. zumindest eine Hydroxylgruppe aufweisen, und
a-d ganze Zahlen sind, die gleich oder unterschiedlich sein können, wobei die Summe der Zahlen a-d zwischen 0 und 200 liegt; 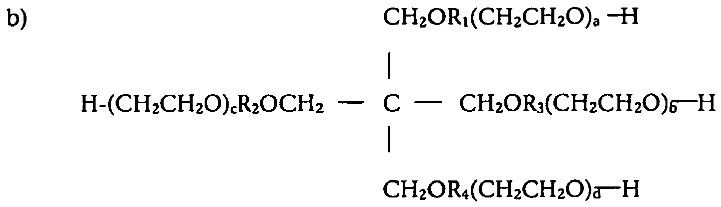
in welcher
R₁₋₄ jeweils eine C₁₂-C₂₆ Fettsäure ist die gleich oder unterschiedlich, gesättigt oder ungesättigt sind, wobei zumindest einer der Reste R₁₋₄ zumindest eine Hydroxylgruppe aufweist,
und
a-d ganze Zahlen sind, die gleich oder unterschiedlich sein können, wobei die Summe der Zahlen a-d zwischen 0 und 200 liegt; 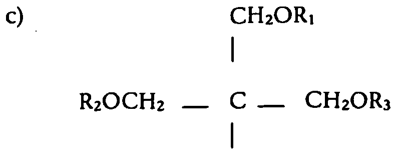 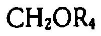
in welcher
R₁₋₄ jeweils eine C₁₂-C₂₆ Fettsäure ist, die gleich oder unterschiedlich, gesättigt oder ungesättigt sind, wobei zumindest an einen der Reste R₁₋₄ mindestens ein hydrophiler Rest aus Homo- oder Copolymeren von Vinylverbindungen geknüpft ist.

8. Verwendung nach einem der Ansprüche 1 bis 6, **dadurch gekennzeichnet, dass** der nichtionische Ester einen HLB-Wert von zwischen 2 und 18, vorzugsweise von zwischen 4 und 14 aufweist.

9. Verwendung nach einem der Ansprüche 6 bis 8, **dadurch gekennzeichnet, dass** der nichtionische Ester ausgewählt ist aus der Gruppe umfassend Polyoxyl-Ricinoleat, Polyoxylglycerol-hydroxystearat, Polyoxylsorbitolhexaoleat.

10. Verwendung nach einem der Ansprüche 1 bis 9, **dadurch gekennzeichnet, dass** die Beschichtungslösung für die hämokopatible Beschichtung aus einer wässrigen Lösung oder Emulsion besteht, die den nichtionischen Ester enthält.

11. Verwendung nach Anspruch 10, **dadurch gekennzeichnet, dass** der nichtionische Ester in der wässrigen Lösung oder Emulsion zu weniger als 2 Gew.-%, vorzugsweise zu weniger als 0,2 Gew.-% enthalten ist.

## Claims

1. A use of at least one nonionic ester in a coating for surfaces coming into contact with blood for improving the hemocompatibility of said surfaces, wherein said nonionic ester is formed from an acyclic C₃-C₆(OH)₃₋₆ polyol and from at least three C₁₂-C₂₆ fatty acids, wherein the ester further includes at least one hydrophilic group.

2. The use of claim 1, **characterized in that** the hydrophilic group of the ester is selected from the group consisting of hydroxyl, methoxyl, ethoxyl and ethoxylate, homopolymers of vinyl compounds and copolymers of vinyl compounds.

3. The use of claims 1 or 2, **characterized in that** the vinyl compound is selected from the group consisting of vinylpyrrolidone, acrylamide, acrylic ester, methacrylic ester.

4. The use of any one of claims 1 to 3, **characterized in that** the at least one hydrophilic group is connected *via* the polyol to the ester.

5. The use of any one of claims 1 to 3, **characterized in that** the at least one hydrophilic group is connected *via* the fatty acid to the ester.

6. The use of any one of claims 1 to 3, **characterized in that** the nonionic ester has a formula which is selected from the following formulae: 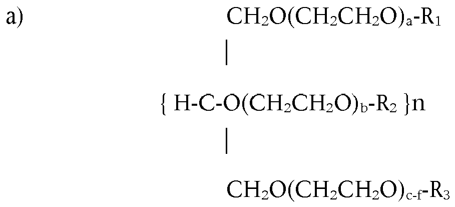 in which
R₁₋₃ is in each case a C₁₂-C₂₆ fatty acid which are identical or different, saturated or unsaturated, and which have where appropriate at least one hydroxyl group,
n is an integer from 1 to 4, and
a-f are integers which may be identical or different, where the total of the numbers a-f is between 0 and 200; 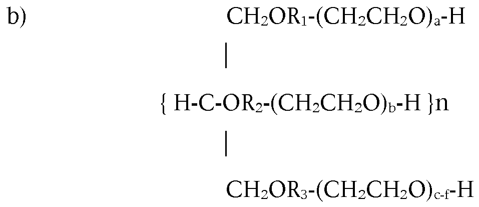
in which
R₁₋₃ is in each case a C₁₂-C₂₆ fatty acid which are identical or different, and saturated or unsaturated, where at least one of the radicals 1-3 has at least one hydroxyl group,
n is an integer from 1 to 4,
a-f are integers which may be identical or different, where the total of the numbers a-f is between 0 to 200; 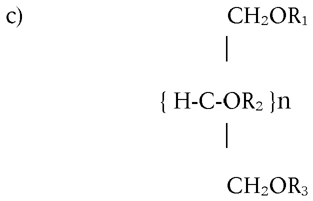
in which
R₁₋₃ is in each case a C₁₂-C₂₆ fatty acid which are identical or different, and saturated or unsaturated, where at least one hydrophilic radical from homo- or copolymers of vinyl compounds is linked to at least one of the radicals R₁₋₃,
n is an integer from 1 to 4.

7. The use of any one of claims 1 to 3, **characterized in that** the nonionic ester has a formula which is selected from the following formulae: 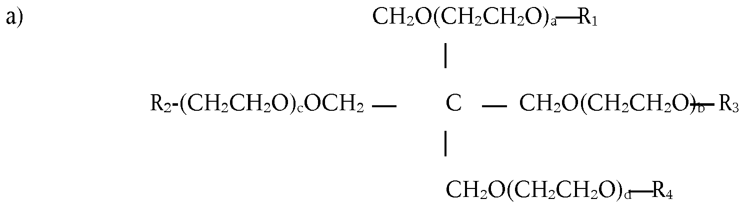 in which
R₁₋₄ is in each case a C₁₂-C₂₆ fatty acid which are identical or different, saturated or unsaturated, and which have where appropriate at least one hydroxyl group, and
a-d are integers which may be identical or different, where the total of the numbers a-d is between 0 and 200; 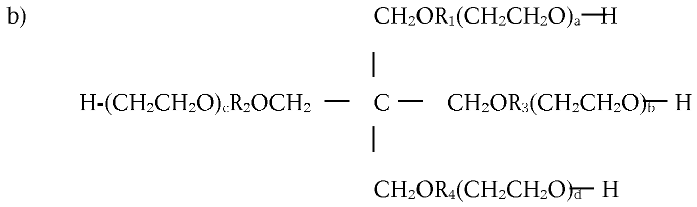
in which
R₁₋₄ is in each case a C₁₂-C₂₆ fatty acid which are identical or different, saturated or unsaturated, where at least one of the radicals R₁₋₄ has at least one hydroxyl group,
and
a-d are integers which may be identical or different, where the total of the numbers a-d is between 0 and 200; 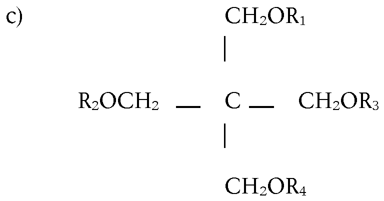
in which
R₁₋₄ is in each case a C₁₂-C₂₆ fatty acid which are identical or different, saturated or unsaturated, where at least one hydrophilic radical from homo- or co-polymers of vinyl compounds is linked to at least one of the radicals R₁₋₄.

8. The use of any one of claims 1 to 6, **characterized in that** the nonionic ester has an HLB value of between 2 and 18, preferably between 4 and 14.

9. The use of any one of claims 6 to 8, **characterized in that** the nonionic ester is selected from the group consisting of polyoxyl ricinoleate, polyoxylglycerol hydroxystearate, polyoxylsorbitol hexaoleate.

10. The use of any one of claims 1 to 9, **characterized in that** the coating solution for the hemocompatible coating is an aqueous solution or emulsion containing the nonionic ester.

11. The use of claim 10, **characterized in that** the aqueous solution or emulsion contains the nonionic ester in an amount of less than 2% by weight, preferably less than 0.2% by weight.

## Revendications

1. Utilisation d'au moins un ester non ionique dans un revêtement destiné à des surfaces entrant en contact avec du sang afin d'améliorer l'hémocompatibilité desdites surfaces, ledit ester non ionique étant formé à partir d'un polyol C₃-C₆(OH)₃₋₆ acyclique et à partir d'au moins trois acides gras en C₁₂-C₂₆, l'ester comprenant en outre au moins un groupe hydrophile.

2. Utilisation selon la revendication 1, **caractérisée en ce que** le groupe hydrophile de l'ester est choisi dans l'ensemble consistant en groupes hydroxyle, méthoxyle, éthoxyle et éthoxylate, homopolymères de composés vinyliques et copolymères de composés vinyliques.

3. Utilisation selon les revendications 1 ou 2, **caractérisée en ce que** le composé vinylique est choisi dans l'ensemble consistant en vinylpyrrolidone, acrylamide, ester acrylique, ester méthacrylique.

4. Utilisation selon l'une quelconque des revendications 1 à 3, **caractérisée en ce que** le ou les groupes hydrophiles sont connectés par l'intermédiaire du polyol à l'ester.

5. Utilisation selon l'une quelconque des revendications 1 à 3, **caractérisée en ce que** le ou les groupes hydrophiles sont connectés par l'intermédiaire de l'acide gras à l'ester.

6. Utilisation selon l'une quelconque des revendications 1 à 3, **caractérisée en ce que** l'ester non ionique a une formule qui est choisie parmi les formules suivantes : 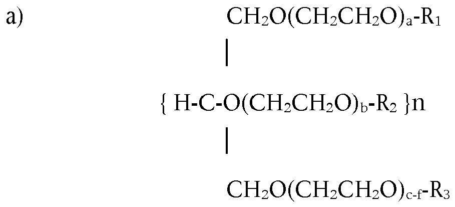 dans laquelle
R₁₋₃ représentent dans chaque cas un acide gras en C₁₂-C₂₆, ceux-ci étant identiques ou différents, saturés ou insaturés, et comprenant, le cas échéant au moins un groupe hydroxyle,
n est un nombre entier de 1 à 4, et
a-f sont des nombres entiers qui peuvent être identiques ou différents, le total des nombres a-f étant compris entre 0 et 200 ; 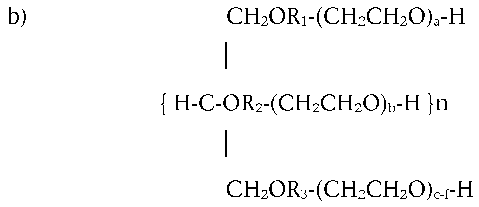
dans laquelle
R₁₋₃ représentent dans chaque cas un acide gras en C₁₂-C₂₆, ceux-ci étant identiques ou différents, saturés ou insaturés, au moins un des radicaux ₁₋₃ contenant au moins un groupe hydroxyle ;
n est un nombre entier de 1 à 4,
a-f sont des nombres entiers qui peuvent être identiques ou différents, le total des nombres a-f étant compris entre 0 et 200 ; 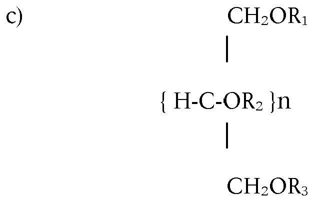
dans laquelle
R₁₋₃ représentent dans chaque cas un acide gras en C₁₂-C₂₆, ceux-ci étant identiques ou différents, saturés ou insaturés, au moins un des radicaux hydrophiles des homo- ou copolymères de composés vinyliques étant lié à au moins un des radicaux R₁₋₃,
n est un nombre entier de 1 à 4.

7. Utilisation selon l'une quelconque des revendications 1 à 3, **caractérisée en ce que** l'ester non ionique a une formule qui est choisie parmi les formules suivantes : 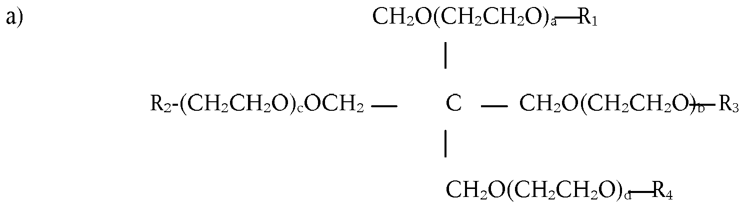 dans laquelle
R₁₋₄ représentent dans chaque cas un acide gras en C₁₂-C₂₆, ceux-ci étant identiques ou différents, saturés ou insaturés, et contenant au moins un groupe hydroxyle, et
a-d sont des nombres entiers qui peuvent être identiques ou différents, le total des nombres a-d étant compris entre 0 et 200 ; 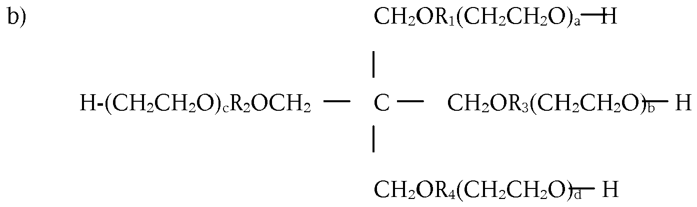
R₁₋₄ représentent dans chaque cas un acide gras en C₁₂-C₂₆, ceux-ci étant identiques ou différents, saturés ou insaturés, au moins un des radicaux R₁₋₄ contenant au moins un groupe hydroxyle,
et
a-d sont des nombres entiers qui peuvent être identiques ou différents, le total des nombres a-d étant compris entre 0 et 200 ; 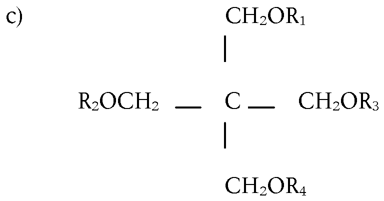
dans laquelle
R₁₋₄ représentent dans chaque cas un acide gras en C₁₂-C₂₆, ceux-ci étant identiques ou différents, saturés ou insaturés, au moins un des radicaux hydrophiles des homo- ou copolymères de composés vinyliques étant lié à au moins un des radicaux R₁₋₄.

8. Utilisation selon l'une quelconque des revendications 1 à 6, **caractérisée en ce que** l'ester non ionique a une valeur de HLB comprise entre 2 et 18, de préférence entre 4 et 14.

9. Utilisation selon l'une quelconque des revendications 6 à 8, **caractérisée en ce que** l'ester non ionique est choisi dans l'ensemble consistant en ricinoléate de polyoxyle, hydroxystéarate de polyoxylglycérol, hexaoléate de polyoxylsorbitol.

10. Utilisation selon l'une quelconque des revendications 1 à 9, **caractérisée en ce que** la solution de revêtement pour le revêtement hémocompatible est une solution ou une émulsion aqueuse contenant l'ester non ionique.

11. Utilisation selon la revendication 10, **caractérisée en ce que** la solution ou l'émulsion aqueuse contient l'ester non ionique en une quantité inférieure à 2 % en poids, de préférence inférieure à 0,2 % en poids.
